# EUROPEAN PATENT APPLICATION

(11) **EP 2 055 196 A1**
(43) Date of publication of application: **06.05.2009**
(21) Application number: 07119479.9
(22) Date of filing: 29.10.2007
(51) Int. Cl.: A23L 1/22, A23L 1/236

(54) **Sweet flavour modulating carboxyalkyl-substituted phenyl derivatives**

(71) Applicant: Givaudan SA, 1214 Vernier (CH)
(72) Inventor: Bouter, Nico, 1261 LK, Blaricum (NL); Koenig, Thorsten, 1411 GP, Naarden (NL); van Ommeren, Esther, 8241 AS, Lelystad (NL); Renes, Harry, 8241 AS, Lelystad (NL); Winkel, Chris, 1402 GL, Bussum (NL)
(74) Representative: Swinkels, Bart Willem

(57) **Abstract**

The present invention relates to the field of flavour modulation in foodstuffs, beverages, tobacco products, oral care products and the like. In particular, the invention relates to substances that are capable of improving, especially enhancing and complementing, sweet flavour impact of other flavour and/or taste imparting substances present in the aforementioned products. The flavour modulating substances of the invention are represented by the following formula (I) 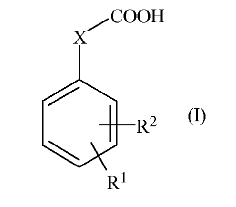 Suitable examples of the present flavour modulating substances include p-coumaric acid, isovanillic acid, 4-methoxycinnamic acid, 3-hydroxy-4-methoxycinnamic acid, 4-methoxymandelic acid, *p*-hydroxy-*m*-methoxymandelic acid, 2-oxo-3-phenyl-propionic acid, piperonylic acid, 4-methoxybenzoic acid, edible salts thereof and edible esters thereof.

## Description

### Field of the Invention

The present invention relates to the field of flavour modulation in foodstuffs, beverages, tobacco products, oral care products and the like. In particular, the invention relates to substances that are capable of improving, especially enhancing and complementing, sweet flavour impact of other flavour and/or taste imparting substances present in the aforementioned products. Thus flavour modulating substances are provided by the present invention as well as compositions containing said substances, methods employing said substances and uses of these substances for improving the flavour of foodstuffs, beverages, tobacco products and the like.

### Background of the Invention

The flavour of foodstuffs and beverages consists of two parts: the aroma and the taste. In general what is perceived through the olfactory epithelium in the nasal cavity is referred to as 'aroma', whereas the term 'taste' is generally used to describe the sensory impact that is perceived via the mouth, especially the tongue. The flavour sensation experienced upon consumption, especially taste, provides the final analysis of food prior to ingestion thereof. Visual and olfactory (smell) signals already give a first indication but only after intake of the food into the mouth the final decision is made either to ingest or to reject the food. Sweet taste is usually a signal that the food is safe (appetising) leading to ingestion of the food. The 'reactions' to salt and umami are really dependent on the strength of the signal. Bitter and sour are usually repulsive taste sensations, leading to rejection. Temperature is another measure by which the food is judged just as well as aching sensations like capsaicin (hot pepper) and certain chemicals (like carbon dioxide).

In short, this means that taste is a very important and very complex system. Until recently most flavour research was focused towards aroma. Especially the last years a series of publications relating to molecules with a (positive) contribution to the taste of foodstuffs has emerged.

Such research has been stimulated significantly by the fact that quite some receptors which are involved in the different taste sensations have been characterized by now (J. Chandrashekar et al., Nature 444, 288 (2006)).

Another interesting aspect of taste is that it can have an impact on aroma. It was reported that people having artificially sweetened water in their mouth were significantly more sensitive to the smell of benzaldehyde than people having plain water in their mouth (P. Dalton et al, Nature Neurosci. 3, 431-432 (2000)).

Several screening systems have been described that make it possible to screen, in a short time, large series of molecules for their (modulating) effect on taste response (cf. WO 04/055048, GB 2396414, WO 01/77292 and US 2004/0072254).

Most research on taste modulation so far has been devoted to taste enhancement in savoury products. Several, mainly Japanese, publications describe umami molecules, i.e. alternatives to mono sodium glutamate (MSG) (H Suzuki et al, J Agric Food Chem 50, 313-318 (2002); K Shima et al, J Agric Food Chem 46, 1465-1468 (1998); Y Ueda et al, Biosc Biotech Biochem 61, 1977 (1997)).

In EP 1291342, a 'general taste enhancer' is disclosed that was reported to be suitable for enhancing sweetness as well.

In patent applications WO 97/04667 and WO 04/075633 tripeptides and amino acid condensates with lactic acid and succinic acid are described that have both their own taste as well as some enhancing properties. Alpha keto acids are reported to give body and mouthfeel to foodstuffs they are added to (US 6,287,620).

Chlorogenic acids are claimed to enhance sweetness and to reduce bitterness (WO 02/100192).

Quite a bit of work has been devoted to find bitter taste suppressors (A.N.Pronin et al, Chemical Senses 29, 583-593 (2004); EP 1401500; P.A. Breslin, Trends in Food Science & Technology 7, 390-399 (1996)).

In sweet and beverage products, further examples of the importance of the gustative dimension of flavourings have been reported. These examples include taste attributes such as bitterness, tingling and cooling-freshness.

US 4,627,987 discloses edible materials comprising a sweetener and a sweetness modifying agent, which agent is capable of increasing the perception of sweetness imparted by the sweetener, typically to reduce the amounts of sweeteners required in the edible materials. The sweetness modifying agent is *meta*-hydroxybenzoic acid. According to US 4,627,987 the pH of the edible material is to be within the range of 2.0-5.5 for the m-hydroxybenzoic acid to have satisfactory effectiveness.

WO 99/15032 discloses the use of 2,4-dihydroxybenzoic acid or a physiologically acceptable salt thereof as a foodstuff sweetener in combination with a significant amount of the artificial sweetener aspartame. It is described in WO 99/15032 that solutions of 2,4-dihydroxybenzoic acid in water are essentially tasteless and that 2,4-dihydroxybenzoic acid increases sweetness in combination with aspartame synergistically, whereas 2,4-dihydroxybenzoic acid does not provide any such advantage when used in combination with other artificial sweeteners, such as Ace-K, alitame or saccharin.

US 2006/0286259 discloses beverage compositions including a flavour component, a sweetener and a sweetener potentiator which includes a combination of 3-hydroxybenzoic acid and 2,4-dihydroxybenzoic acid. US 2006/0286259 furthermore describes the use of hydrophobic sweeteners, for example 2-acetyl benzoic acid.

GB 2 157 148 discloses substances that have the capability of reducing the sweetness of an ingestible product containing a sweetening sugar or sugar alcohol, thereby 'enhancing' the flavour of such products. These substances do not truly enhance the impact of other flavour and/or taste imparting substances, rather they reduce the overwhelming sweetness in products wherein the inclusion of high amounts of sugars is required for other reasons than their impact on taste.

There is still a need for substances having a positive contribution to the flavour, especially taste, of foodstuffs, beverages, tobacco products and/or oral care products they are incorporated in. One objective of the present invention is to provide such substances as well as compositions comprising them that can complement and improve the impact of flavour and/or taste imparting substances, in particular that can enhance the impact of sweet flavour and/or taste imparting substances.

### Summary of the Invention

The present inventors have surprisingly found that substances selected from the group represented by formula (I): 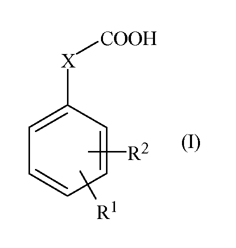 and edible salts thereof and edible esters thereof can advantageously be used for modulating the flavour of foodstuffs, beverages, tobacco products and oral care products, especially for enhancing sweet taste impact of other flavour and/or taste imparting substances comprised in such products.

The present flavour modulating substances can be used to impart desirable taste attributes in a wide variety of applications and products. In addition, the present flavour modulating substances are capable of modulating the taste and/or aroma impact of other flavour and/or taste imparting substances contained within these same products, thereby improving the overall flavour quality of these products.

Anisic acid and cinnamic acid are substances that are abundant in certain natural materials as well as in extracts thereof that have traditionally been used for their specific flavour characteristics. These substances, which are encompassed by the above formula (I) have, however, never been suggested to enhance the flavour impact or flavour perception of other flavour and/or taste imparting substances.

EP 0 125 049 discloses sweetness inhibitors which can be used to reduce the sweetness of sucrose, especially were it is not used for its sweetening power but e.g. as a structural ingredient, for its antimicrobial action or as a carbohydrate source. Some of the sweetness inhibitors disclosed in EP 0 125 049 structurally resemble the substances of the present invention; EP 0 125 049 specifically discloses the substance phenyl pyruvic acid and suggests it has utility as a sweetness inhibitor. EP 0 125 049 does actually exemplify the application of phenylpyruvic acid as a sweetness inhibitor in a food product or test composition. EP 0 125 049 does not mention or suggest any taste or flavour characteristic other than sweetness inhibition, of substances encompassed by or resembling the above formula.

WO 00/69282 discloses compositions comprising neotame (N-[N-(3,3-dimethylbutyl)-L-α-aspartyl]-L-phenylalanine methyl ester) in combination with a hydrophobic acid additive and/or salts of neotame and such a hydrophobic acid additive. According to WO 00/69282 any hydrophobic acid additive or neotame salt thereof will effect at least one characteristic of the taste of neotame in a product, although it is acknowledged by the inventors of WO 00/69282 that not all combinations will be effective in all products conceivable. Since the entire group of hydrophobic acid additives disclosed in WO 00/69282 do not bear any structural resemblance other than being hydrophobic and containing an acidic moiety and since WO 00/69282 mainly refers to the dissolution rate and the solubility of neotame, it is clear to the skilled person that the invention of WO 00/69282 concerns modification of the physicochemical properties of neotame compositions. The specific teachings of WO 00/69282 would not lead the skilled person to the conclusion that any of the hydrophobic acid additives could be effective in combination with any other type of sweetener, let alone any other type of flavour and/or taste imparting substance. Moreover, WO 00/69282 does not provide any teachings or suggestions to the effect that any of the hydrophobic acid additives themselves actually interact with taste receptors in the epithelium of the oral cavity and/or with the olfactory epithelium of the nasal cavity such as to modify the perceived flavour impact of other flavour and/or taste imparting substances.

Thus, the present invention, for the first time, provides the use of substances represented by the aforementioned formula (I) for improving the flavour perception of flavour and/or taste imparting substances other than N-[N-(3,3-dimethylbutyl)-L-α-aspartyl]-L-phenylalanine methyl ester.

Therefore, the present invention relates to flavour compositions comprising these flavour modulating substances in combination with one or more of such flavour and/or taste imparting substances. Furthermore, methods of improving the flavour of a product selected from foodstuffs, beverages, tobacco products and oral care products, as well as products comprising the present flavour modulating substances are provided by the invention. In a further aspect the use of the present flavour modulating substances for enhancing sweet taste impact of other flavour and/or taste imparting substances is provided. In yet a further aspect, the invention concerns specific flavour modulating substances represented by the aforementioned formula (I) that have not been described or disclosed previously.

### Detailed Description of the Invention

Hence one aspect of the invention concerns the use of one or more flavour modulating substances selected from the group consisting of substances according to formula (I), edible salts thereof and edible esters thereof: 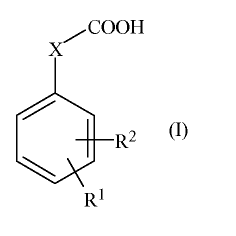 wherein X represents a covalent bond or -X-COOH represents a moiety selected from -CH=CH-COOH, -CH₂-CO-COOH and -CHOH-COOH; R¹ represents hydrogen, hydroxyl or lower alkoxyl; R² represents hydrogen, hydroxyl or lower alkoxyl; or R¹ and R² together form a 4, 5 or 6 membered heterocyclic moiety comprising at least 1 heteroatom selected from oxygen and sulphur; wherein at least one of X, R¹ and R² comprises one or more carbon atoms; for enhancing sweet flavour impact of flavour and/or taste imparting substances in a product selected from the group of foodstuffs, beverages, oral care products and tobacco products, wherein the product does not comprise a sweetening effective amount of N-[N-(3,3-dimethylbutyl)-L-α-aspartyl]-L-phenylalanine methyl ester.

A further aspect of the invention concerns a flavour composition comprising at least 0.5 wt% of one or more flavouring substances in addition to at least 10 ppm of one or more flavour modulating substances selected from the group consisting of substances according to formula (I), edible salts thereof and edible esters thereof, wherein X represents a covalent bond or -X-COOH represents a moiety selected from -CH=CH-COOH, -CH₂-CO-COOH and -CHOH-COOH; R¹ represents hydrogen, hydroxyl or lower alkoxyl; R² represents hydrogen, hydroxyl or lower alkoxyl; or R¹ and R² together form a 4, 5 or 6 membered heterocyclic moiety comprising at least 1 heteroatom selected from oxygen and sulphur; wherein at least one of X, R¹ and R² comprises one or more carbon atoms; with the exception of phenylpyruvic acid, anisic acid and cinnamic acid and salts and esters thereof, and with the proviso that the flavour composition does not comprise a sweetening effective amount of N-[N-(3,3-dimethylbutyl)-L-α-aspartyl]-L-phenylalanine methyl ester

Yet a further aspect of the invention concerns novel flavour modulating substances selected from the group consisting of substances according to formula (I), edible salts thereof and esters thereof, wherein -X-COOH represents a moiety selected from -CH₂-CO-COOH and -CHOH-COOH; R¹ represents hydrogen, hydroxyl or lower alkoxyl; R² represents hydrogen, hydroxyl or lower alkoxyl; or R¹ and R² together form a 4, 5 or 6 membered heterocyclic moiety comprising at least 1 oxygen atom, with the exception of mandelic acid and phenylpyruvic acid and salts and esters thereof.

The term "lower" as used herein in connection to "alkoxyl" means that the moiety concerned comprises a carbon chain portion of not more than six carbon atoms, preferably of not more than four carbon atoms, most preferably of not more than two carbon atoms. In a preferred embodiment the lower alkoxyl is a branched or unbranched saturated C₁-C₆ alkoxyl, preferably C₁-C₄ alkoxyl, most preferably C₁-C₂ alkoxyl.

The present inventors have found that the above-defined flavour modulating substances are very useful flavour ingredients which, particularly in the presence of other flavour and/or taste imparting substances, are capable of imparting highly appreciated taste sensations to the products in which they are incorporated, specifically "vanilla" and/or "sweet". Because of this, the present substances can be employed to improve the flavour, especially taste, including "mouthfeel" and "body", of foodstuffs, beverages, tobacco products and oral care products.

The flavour modulating substances of the present invention as such are capable of imparting highly desirable taste attributes. In addition, it has been found that the flavour modulating substances according to the invention are capable of complementing and modifying the sensory impact of other, flavour and/or taste imparting substances, contained in the aforementioned products, especially of enhancing and modulating "sweet taste impact" thereof.

Throughout this document the term "flavour" is used to describe the sensory impact that is perceived via the mouth, especially the tongue, and the olfactory epithelium in the nasal cavity. The term "complementing and modifying the sensory impact" as used herein refers to the capability of the present compositions or substances to alter the taste and/or aroma impact of other, flavour and/or taste imparting, substances present within the same product, with the proviso that this change in taste impact is not caused by the flavour contribution of said composition or substance *per se*, but instead that it mainly results from the combined effect of on the one hand the present flavour modulating composition or substance and on the other hand the other flavour and/or taste imparting substance(s). The present flavour modulating substances combine the capability of modulating the taste and/or aroma of other, flavour and/or taste imparting, substances and a taste contribution of their own. The favourable impact of the present flavour modulating substances is believed to be the result of the combination of these two effects.

Because the flavour modulating substances according to the invention are not particularly volatile, they do not produce a strong aroma impact, even though they can affect the aroma impact of other flavour imparting substances. Here the term "aroma" refers to the aspect of flavour that is perceived through the olfactory epithelium. Because of the low volatility of the present flavour modulating substances it is believed that the advantageous properties of these substances are somehow associated with the interaction that these substances have on the sensory receptors located within the mouth.

It was found that particularly satisfying results were obtained with flavour modulating substances of the invention wherein, -X-COOH represents a moiety selected from the group of -CH=CH-COOH, -CH₂-CO-COOH and -CHOH-COOH.

It was found that particularly satisfying results were obtained with flavour modulating substances in accordance with the present invention, wherein R¹ represents a substituent that is attached to the phenyl ring in the *para* position, i.e. to carbon atom 4, relative to the -X-COOH moiety. According to a particularly preferred embodiment of the present invention R¹ represents hydrogen, *p*-hydroxy, *p*-methoxy , more preferably *p*-hydroxy or *p*-methoxy, most preferably *p*-methoxy.

In yet another, equally preferred embodiment of the invention, R² represents hydrogen, hydroxyl, methoxy or ethoxy, more preferably hydrogen, hydroxyl or methoxy, more preferably hydrogen or hydroxyl, most preferably hydrogen. Furthermore, it is particularly preferred that R² is attached to the phenyl ring in the *meta*-position.

In yet another equally preferred embodiment of the invention R¹ and R² together with two carbon atoms of the phenyl ring form a 5 mebered heterocycle comprising at least 1, preferably 2, oxygen atoms. Most preferably R¹ and R² together represent a moiety having the formula -O-CH₂-O-. In addition, it is particularly preferred that R¹ and R² are attached to neighbouring carbon atoms of the phenyl ring, preferably to carbon atoms numbered 3 and 4, the carbon atom substituted with the -X-COOH moiety being designated as carbon atom 1.

In an even more preferred embodiment, the invention relates to flavour modulating substances selected from the group of *p*-coumaric acid, isovanillic acid, 4-methoxycinnamic acid, 3-hydroxy-4-methoxycinnamic acid, 4-methoxymandelic acid, 4-hydroxy-3-methoxymandelic acid, *p*-hydroxy-*m*-methoxymandelic acid, 2-oxo-3-phenyl-propionic acid, piperonylic acid, 4-methoxybenzoic acid, edible salts thereof and edible esters thereof. More preferably the invention relates to flavour modulating substances selected from the group of *p*-coumaric acid, 4-methoxycinnamic acid, 3-hydroxy-4-methoxycinnamic acid, 4-methoxymandelic acid, 4-hydroxy-3-methoxymandelic acid, *p*-hydroxy-*m*-methoxymandelic acid, 2-oxo-3-phenyl-propionic acid, piperonylic acid, edible salts thereof and edible esters thereof.

As used herein the term 'edible esters thereof' refers to a derivative of a flavour modulating substance of the invention and an acid or alcohol formed by reaction of said acid or alcohol with a hydroxyl group or carboxyl group, respectively, that is present in said flavour modulating substance, said derivative being suitable for human consumption, i.e. being non-toxic, and having flavour modulating properties in accordance with was has been explained herein before.

The term "edible salt", as used herein, refers to a salt that is generally considered suitable for human consumption, particularly a non-toxic salt. Acceptable salts include base addition salts and acid addition salts of the corresponding free acid. These salts typically may be prepared by conventional means from the free acid of the present flavour modulating substances. Illustrative base addition salts include metallic salts and organic salts. Metallic salts include alkali metal salts, such as sodium and potassium salts, alkaline earth metal salts, such as calcium and magnesium salts. Organic salts include salts made from secondary, tertiary and quaternary amines such as diethylamine, triethylamine, ethanolamine, diethanolamine, and salts made from cationic amino acids such as arginine, lysine and histidine. Examples of suitable acid addition salts include hydrochloride, phosphate, hydrogen phosphate, acetate, aspartate, ascorbate, citrate, gluconate, lactate, succinate, tartrate, etc.

The term "comprising" whenever used in this document is intended to indicate the presence of stated features, integers, steps, components, but not to preclude the presence or addition of one or more other features, integers, steps, components or groups thereof.

As specified above, the flavour compositions of the present invention do not comprise a sweetening effective amount of N-[N-(3,3-dimethylbutyl)-L-α-aspartyl]-L-phenylalanine methyl ester. As used herein, "a sweetening effective amount" refers to an amount that upon dosing the flavour composition does not produce an increase in sweetness as compared to an otherwise identical flavour composition that is free ofN-[N-(3,3-dimethylbutyl)-L-α-aspartyl]-L-phenylalanine methyl ester. Neotame's threshold sweetening level has been determined to be approximately 4 ppm. In a particularly preferred embodiment of the invention, the present flavour compositions therefore comprise less than 40 ppm of N-[N-(3,3-dimethylbutyl)-L-α-aspartyl]-L-phenylalanine methyl ester, more preferably less than 20 ppm, more preferably less than 4 ppm, most preferably, the flavour compositions of the invention do not contain N-[N-(3,3-dimethylbutyl)-L-α-aspartyl]-L-phenylalanine methyl ester.

In another preferred embodiment, flavour compositions as defined before are provided, wherein the molar ratio of flavour modulating substances according to the invention to N-[N-(3,3-dimethylbutyl)-L-α-aspartyl]-L-phenylalanine methyl ester is higher than 2, preferably it is higher than 3, more preferably it is higher than 5, more preferably it is higher than 10, most preferably it is higher than 50.

In a particularly preferred embodiment of the invention, a flavour composition as defined herein before is provided, wherein said one or more flavour modulating substances are present in an amount of at least 10 ppm, more preferably at least 100 ppm, most preferably at least 1000 ppm. Typically the flavour compositions of the present inventions comprise the one or more flavour modulating substances in an amount of less than 100,000 ppm, more preferably of less than 10,000 ppm, most preferably less than 5,000 ppm.

The expression "ppm" as used herein refer to amounts expressed in parts per million, 1 ppm corresponding to 1 mg/kg. This expression is a common term in the art of flavours and fragrances and understood by the skilled person as having the meaning given here.

As used herein the term "flavouring substance" is meant to encompass any food grade substance that is capable of imparting a detectable flavour impact, typically at concentrations below 1 wt.%, more preferably below 0.1 wt.%. Suitable examples of flavouring substances include alcohols, aldehydes, ketones, esters, ethers, acetates, nitriles, terpene hydrocarbons, nitrogenous or sulphurous heterocyclic compounds and essential oils, and said flavouring substances can be of natural or synthetic origin. Many of these are listed in reference texts such as the book by S. Arctander, Perfume and Flavor Chemicals, 1969, Montclair, New Jersey, USA, or its more recent versions, or in other works of a similar nature, as well as in the abundant patent literature in the field of flavours. It will be clear to the skilled person that the type of flavouring substance added would entirely depend on the type of product to which the composition is added.

In a preferred embodiment the flavour compositions according to the invention comprises one or more flavouring substances in an amount of at least 0.5 wt%, preferably at least 1 wt%, most preferably at least 2 wt%, based on the total weight of the composition. Typically the amount of flavouring substances comprised in the present flavour compositions is below 10 wt%, preferably it is below 5 wt%.

Typically, in the present flavour composition the taste modulating substances and flavouring substances as defined herein before are employed in a weight ratio of less than 1:50, preferably less than 1:100, more preferably less than 1:1,000 and most preferably in a weight ratio of less than 1:10,000.

In a particularly preferred embodiment, the present flavour composition comprises sweet taste imparting substances, especially natural or artificial sweeteners. Suitable examples of natural sweeteners include sucrose, fructose, glucose, high fructose corn syrup, xylose, arabinose, and rhamnose, the sugar alcohols erythritol, xylitol, mannitol, sorbitol, and inositol. Suitable examples of artificial sweeteners include AceK, aspartame, sucralose, and saccharine. These sweet taste imparting substances may be included in any suitable amount. Since the sweet taste imparting effect of these substances will be enhanced by the presence of the flavour modulating substances of the invention, said amounts may typically be lower than those conventionally applied. Typically an amount will be included which is equivalent in sweetness to at least 0.5 wt% of sucrose.

The flavour composition according to the present invention may suitably be prepared in the form of a liquid, a paste or a powder. In a particularly preferred embodiment the flavour composition is a free flowing powder. Typically the present flavouring compositions further comprise at a flavour carrier material. As used herein, the term flavour "carrier material" encompasses a bulk material that is practically neutral from a flavour point of view, i.e. which does not significantly alter the organoleptic properties of flavouring ingredients. Said carrier may be a liquid or a solid. Suitable examples of liquid carriers include emulsifying systems, i.e. a solvent and a surfactant system, or a solvent commonly used in flavours. As suitable no-limiting examples of solvents commonly used in flavours, one can cite compounds such as propylene glycol, triacetine, triethyl citrate, ethanol, vegetable oils or terpenes. Examples of solid carriers include absorbing gums or polymers or encapsulating materials. Examples of such materials, for example, may comprise wall-forming and plasticizing materials, such as mono, di- or trisaccharides, natural or modified starches, hydrocolloids, cellulose derivatives, polyvinyl acetates, polyvinylalcohols, proteins or pectins, maltodextrins or materials cited in reference texts such as H. Scherz, Hydrokolloids: Stabilisatoren, Dickungs- und Gehermittel in Lebensmittel, Band 2 der Schriftenreihe Lebensmittelchemie, Lebensmittelqualitat, Behr's VerlagGmbH & Co., Hamburg, 1996.

Typical examples of flavour compositions according to the present invention include dairy flavourings and sweet flavourings.

A further aspect of the invention relates to a product selected from the group of foodstuffs, beverages, oral care products and tobacco products, preferably from the group of foodstuffs and beverages, said product comprising a flavour modulating amount of one or more of the present flavour modulating substances selected from the group of substances represented by formula (I), edible salts thereof and edible esters thereof, wherein -X-COOH represents a moiety selected from -CH=CH-COOH, -CH₂-CO-COOH and -CHOH-COOH; R¹ represents hydrogen, hydroxyl or lower alkoxyl; R² represents hydrogen, hydroxyl or lower alkoxyl; or R¹ and R² together form a 4, 5 or 6 membered heterocyclic moiety comprising at least 1 heteroatom selected from oxygen and sulphur; with the proviso that said flavour modulating substance is not phenylpyruvic acid, anisic acid, cinnamic acid or a salt or ester thereof and wherein said product does not comprise a sweetening effective amount of N-[N-(3,3-dimethylbutyl)-L-α-aspartyl]-L-phenylalanine methyl ester.

Typically, the present product comprises at least 0.01 ppm of one or more of the present flavour modulating substances. More preferably, products of the invention comprise at least 0.1 ppm, more preferably at least 1 ppm, most preferably at least 5 ppm of one or more of the flavour modulating substances according to formula (I) or edible salts or esters thereof. Typically, the aforementioned products preferably contain the flavour modulating substances in a concentration of not more than 10,000 ppm, preferably of not more than 1,000 ppm, more preferably of not more than 500 ppm, most preferably of not more than 100 ppm. The precise level in which the present substances are incorporated depends on the nature of the flavour modulating substance(s) and the nature of the product, as will be clear to the skilled person, and as will be illustrated in the examples.

As specified above, the products of the present invention do not comprise a sweetening effective amount of N-[N-(3,3-dimethylbutyl)-L-α-aspartyl]-L-phenylalanine methyl ester. In a particularly preferred embodiment of the invention, the present flavour compositions comprise less than 4 ppm of N-[N-(3,3-dimethylbutyl)-L-α-aspartyl]-L-phenylalanine methyl ester, more preferably less than 2 ppm, more preferably less than 1 ppm, most preferably, the products of the invention do not contain N-[N-(3,3-dimethylbutyl)-L-α-aspartyl]-L-phenylalanine methyl ester.

In another preferred embodiment, products as defined before are provided, wherein the molar ratio of flavour modulating substances according to the invention to N-[N-(3,3-dimethylbutyl)-L-α-aspartyl]-L-phenylalanine methyl ester is higher than 2, preferably it is higher than 3, more preferably it is higher than 5, more preferably it is higher than 10, most preferably it is higher than 50.

In a particularly preferred embodiment, the present product comprises sweet taste imparting substances, especially natural or artificial sweeteners. Suitable examples of natural sweeteners include sucrose, fructose, glucose, high fructose corn syrup, xylose, arabinose, and rhamnose, the sugar alcohols erythritol, xylitol, mannitol, sorbitol, and inositol. Suitable examples of artificial sweeteners include AceK, aspartame, sucralose, and saccharine. These sweet taste imparting substances may be included in any suitable amount. Since the sweet taste imparting effect of these substances will be enhanced by the presence of the flavour modulating substances of the invention, said amounts may typically be lower than those conventionally applied. Typically an amount will be included which is equivalent in sweetness to 0.5-7 wt% of sucrose.

Preferred examples of foodstuffs according to the present invention include dairy products, such as yoghurts and ice creams, confectionery products, pastry products, bread products, baker's products, cereal products, rice products, tapioca products, 'sago products, biscuit products, desert products, jellies, jams and the like.

Preferred examples of beverages according to the present invention include soft drinks, dairy drinks, fruit juices and alcoholic beverages.

The term 'tobacco products', as used herein, refers to any type of tobacco product for smoking applications as well as for non-smoking applications. It is furthermore noted that tobacco-like products are available for both smoking and non-smoking applications. The use of the present flavour modulating substances in tobacco substitutes is also encompassed by the present invention.

Suitable examples of oral care products according to the present invention include toothpastes, mouthwashes, dental floss, anti-plaque and anti-gingivitis compositions.

Most preferably, in accordance with the invention the product is selected from the group of foodstuffs and beverages.

A further aspect of the invention relates to a method of improving the flavour of a product selected from the group of foodstuffs, beverages, oral care products and tobacco products, said method comprising incorporating in said product a flavour modulating amount of one or more of the present flavour modulating substances selected from the group of substances represented by formula (I), edible salts thereof and edible esters thereof, wherein wherein X represents a covalent bond or -X-COOH represents a moiety selected from -CH=CH-COOH, -CH₂-CO-COOH and -CHOH-COOH; R¹ represents hydrogen, hydroxyl or lower alkoxyl; R² represents hydrogen, hydroxyl or lower alkoxyl; or R¹ and R² together form a 4, 5 or 6 membered heterocyclic moiety comprising at least 1 heteroatom selected from oxygen and sulphur; wherein at least one of X, R¹ and R² comprises one or more carbon atoms with the exception of phenylpyruvic acid, anisic acid, cinnamic acid and salts and esters thereof, wherein said product does not comprise a sweetening effective amount of N-[N-(3,3-dimethylbutyl)-L-α-aspartyl]-L-phenylalanine methyl ester.

Particularly preferred examples and embodiments of the flavour modulating substances, the products and amounts in accordance with this aspect of the invention are as described here above in relation to the other aspects of the invention.

As mentioned here above, the present flavour modulating substances are particularly suitable for modulating, especially improving and/or complementing sweet taste impact. Hence a preferred embodiment of the invention relates to the afore defined use for improving and/or complementing sweet taste impact.

In yet a further aspect of the present invention, novel flavour modulating substances according to formula (I) as well as salts and esters thereof are provided,wherein -X-COOH represents a moiety selected from -CH₂-CO-COOH, and - CHOH-COOH; R¹ represents hydrogen, hydroxyl or lower alkoxyl; R² represents hydrogen, hydroxyl or lower alkoxyl; or R¹ and R² together form a 4, 5 or 6 membered heterocyclic moiety comprising at least 1 oxygen atom; with the exception of phenylpyruvic acid and mandelic acid and salts and esters thereof.

Particularly preferred embodiments of the flavour modulating substance of this aspect of the invention are in accordance with those described here above in relation to the other aspects of the invention. Most preferably the novel flavour modulating substances in accordance with the invention is selected from the group of 4-methoxymandelic acid and 2-oxo-3-phenyl-propionic acid.

It is within the skills of the trained professional to design synthesis routes to prepare any novel substance provided by the present invention. Different routes may be available for producing a given substances, some of which may be more convenient and efficient than others.

The present invention and its preferred embodiments are further illustrated in the following examples, which are not intended to limit the scope in any way.

### Examples

### Example 1:

Full fat milk (3% fat) was sweetened with 5% sugar (sucrose). To one part of the sweet milk was added: 10 ppm 4 methoxy cinnamic acid and 10 ppm p coumaric acid. The milk flavoured in accordance with the present invention was tasted and compared with the reference by a group of experienced flavourists. The flavoured sample was described as:
- being more sweet than the reference sample;
- having full body as compared to the reference sample; and
- having more mouthfeel than the reference sample.

### Example 2:

A yoghurt drink containing 2% fat was sweetened with 6% sugar (sucrose). To one part of the sweetened yoghurt drink 20 ppm of p-hydroxy-m-methoxy-mandelic acid was added. The yoghurt flavoured in accordance with the present invention was tasted and compared with the reference by a group of experienced flavourists. The flavoured sample was described as:
- being more sweet than the reference sample; and
- being more vanilla-like than the reference sample.

### Example 3:

Ice cream (containing 12 % fat and 6% sugar (sucrose)) was prepared and split into two parts. To one part of the ice cream 10 ppm 2-oxo-3-phenyl-propionic acid was added in accordance with the present invention. The ice cream sample in accordance with the invention was tasted and compared with the reference sample by a group of experienced flavourists. Said flavoured sample was described as:
- being more sweet than the reference sample;
- having 'rummy' character as compared to the reference sample; and
- having 'fruity' character as compared to the reference sample.

## Claims

1. Use of one or more flavour modulating substances selected from the group consisting of substances according to formula (I), edible salts thereof and edible esters thereof: 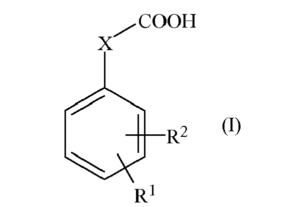 wherein X represents a covalent bond or -X-COOH represents a moiety selected from -
CH=CH-COOH, -CH₂-CO-COOH and -CHOH-COOH;
R¹ represents hydrogen, hydroxyl or lower alkoxyl;
R² represents hydrogen, hydroxyl or lower alkoxyl; or
R¹ and R² together form a 4, 5 or 6 membered heterocyclic moiety comprising at least 1 heteroatom selected from oxygen and sulphur;
wherein at least one of X, R¹ and R² comprises one or more carbon atoms; for enhancing sweet flavour impact of flavour and/or taste imparting substances in a product selected from the group of foodstuffs, beverages, oral care products and tobacco products, wherein the product does not comprise a sweetening effective amount of N-[N-(3,3-dimethylbutyl)-L-α-aspartyl]-L-phenylalanine methyl ester.

2. Use according to claim 1, wherein the product does not comprise N-[N-(3,3-dimethylbutyl)-L-α-aspartyl]-L-phenylalanine methyl ester.

3. Flavour composition comprising at least 0.5 wt% of one or more flavouring substances in addition to at least 10 ppm of one or more flavour modulating substances selected from the group consisting of substances according to formula (I), edible salts thereof and edible esters thereof:
wherein X represents a covalent bond or -X-COOH represents a moiety selected from - CH=CH-COOH, -CH₂-CO-COOH and -CHOH-COOH;
R¹ represents hydrogen, hydroxyl or lower alkoxyl;
R² represents hydrogen, hydroxyl or lower alkoxyl; or
R¹ and R² together form a 4, 5 or 6 membered heterocyclic moiety comprising at least 1 heteroatom selected from oxygen and sulphur;
wherein at least one of X, R¹ and R² comprises one or more carbon atoms with the exception of phenylpyruvic acid, anisic acid and cinnamic acid and salts and esters thereof, and with the proviso that the flavour composition does not comprise a sweetening effective amount of N-[N-(3,3-dimethylbutyl)-L-α-aspartyl]-L-phenylalanine methyl ester.

4. Flavour composition according to claim 3, wherein the flavour composition does not comprise N-[N-(3,3-dimethylbutyl)-L-α-aspartyl]-L-phenylalanine methyl ester.

5. Flavour composition according to claim 3 or 4, wherein -X-COOH represents a moiety selected from the group of -CH=CH-COOH and -CHOH-COOH.

6. Flavour composition according to any one of claims 3-5, wherein R¹ is in the *para* position.

7. Flavour composition according to any one of claims 3-6, wherein R¹ and R² are independently selected from hydrogen, hydroxyl, methoxyl and ethoxyl or wherein R¹ and R² together represent a moiety having the formula -O-CH₂-O-.

8. Flavour composition according to any one of claims 3-7, wherein the flavour modulating substance is selected from the group of p-coumaric acid, isovanillic acid, 4-methoxycinnamic acid, 3-hydroxy-4-methoxycinnamic acid, 4-methoxymandelic acid, *p*-hydroxy-*m*-methoxymandelic acid, 2-oxo-3-phenyl-propionic acid, piperonylic acid, 4-methoxybenzoic acid, edible salts thereof and edible esters thereof.

9. Flavour composition according to any one of claims 3-8, wherein said one or more flavour modulating substances are present in an amount of at least 100 ppm.

10. Product selected from the group of foodstuffs, beverages, oral care products and tobacco products, said product comprising a flavour modulating amount of one or more flavour modulating substances as defined in any one of claims 3-8 and wherein the product does not comprise a sweetening effective amount of N-[N-(3,3-dimethylbutyl)-L-α-aspartyl]-L-phenylalanine methyl ester.

11. Product according to claim 10, wherein said flavour modulating amount is an amount of at least 0.01 ppm.

12. Method of improving the flavour of a product selected from the group of foodstuffs, beverages, oral care products and tobacco products, said method comprising incorporating in said product a flavour modulating amount of one or more one or more flavour modulating substances as defined in any one of claims 3-8, and wherein the product does not comprise a sweetening effective amount of N-[N-(3,3-dimethylbutyl)-L-α-aspartyl]-L-phenylalanine methyl ester.

13. Method according to claim 12, wherein said flavour modulating amount is an amount of at least 0.01 ppm.

14. Flavour modulating substance selected from the group consisting of substances according to formula (I), edible salts thereof and esters thereof: 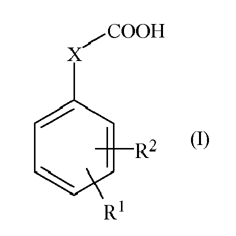 wherein -X-COOH represents a moiety selected from -CH₂-CO-COOH and -CHOH-COOH;
R¹ represents hydrogen, hydroxyl or lower alkoxyl;
R² represents hydrogen, hydroxyl or lower alkoxyl; or
R¹ and R² together form a 4, 5 or 6 membered heterocyclic moiety comprising at least 1 oxygen atom, with the exception of phenylpyruvic acid and mandelic acid and salts and esters thereof.
